# EUROPÄISCHE PATENTSCHRIFT

(11) **EP 2 419 636 B1**
(45) Veröffentlichungstag und Bekanntmachung des Hinweises auf die Patenterteilung: **25.09.2013**
(21) Anmeldenummer: 10718853.4
(22) Anmeldetag: 14.04.2010
(51) Int. Cl.: F04B 43/08

(54) **VERFAHREN UND VORRICHTUNG ZUR PULSATIONSFREIEN VOLUMETRISCHEN FÖRDERUNG VON FLUIDEN UND SUSPENSIONEN**
METHOD AND APPARATUS FOR THE PULSATION-FREE VOLUMETRIC DELIVERY OF FLUIDS AND SUSPENSIONS
PROCÉDÉ ET DISPOSITIF POUR LE TRANSPORT VOLUMÉTRIQUE SANS PULSATION DE FLUIDES ET SUSPENSIONS

(30) Priorität: 17.04.2009 DE 102009017918
(43) Veröffentlichungstag der Anmeldung: 22.02.2012
(73) Patentinhaber: Fachhochschule Jena, 07745 Jena (DE)
(72) Erfinder: SCHIMMELPFENNIG, Michael, 99423 Weimar (DE); FELLER, Karl-Heinz, 07745 Jena (DE)
(74) Vertreter: Oehmke, Volker
(86) Internationale Anmeldenummer: PCT/DE2010/050019
(87) Internationale Veröffentlichungsnummer: WO 2010/118740

(56) Entgegenhaltungen:
- EP-A1- 0 409 001
- WO-A2-2004/076859
- DE-A1- 10 118 086
- DE-A1- 19 509 242
- DE-A1-102008 017 346
- US-A- 5 217 355

## Beschreibung

Die Erfindung bezieht sich auf ein Verfahren sowie eine Vorrichtung zur pulsationsfreien volumetrische Förderung von Fluiden und Suspensionen.

Bei vielen labortechnischen und medizinischen Anwendungen werden Pumpen benötigt, die einerseits exakt volumetrisch dosierbar sind, andererseits aber auch konstante Förderraten aufweisen. Nur so sind beispielsweise zeitlich stabile Mischprozesse mit mehreren Pumpen, die gleichzeitige Gabe mehrerer intravenöser Infusionen oder die gleichförmige Umwälzung in volumenstarren Fluid-Kreisläufen realisierbar.

Pumpsysteme weisen im Allgemeinen eine periodische Fördercharakteristik auf. Die für die einzelnen Förderbewegungen (Saug- und Druckhübe) notwendige Umkehr der relativen Bewegungsrichtung der verdrängenden Bauteile, wie z. B. Kolben oder Stößel, einer Pumpanordnung lassen den Betrag des Förderstromes schwanken. Im ungünstigsten Fall kommt es zum Abreisen der Strömung und zu ungewollten Effekten wie der Entstehung von lokalen Unterdrücken. Solche Kavitationen können mehrere schädliche Wirkungen entfalten. Auftretende Vibrationen verstärken zum einen die Arbeitsgeräusche der Pumpanordnung und können zum anderen die Lebensdauer einer Pumpe und deren Wartungsintervalle negativ beeinflussen. Des Weiteren ist gerade beim Zusammenführen von Fluidströmen, wie zum Beispiel beim Mischprozess, ein gleich bleibendes Verhältnis des Gesamtvolumenstromes und somit auch der einzelnen Stoffströme entscheidend. Nur so kann eine zeitlich konstante Mischung gewährleistet werden.

Aus der Schrift DD 117 108, welche als nächstkommender Stand der Technik angesehen werden kann, ist bekannt, dass sich Druckpulsationen durch den gleichzeitigen Einsatz mehrerer Pumpen bzw. durch den gleichzeitigen Einsatz mehrerer Pumpelemente reduzieren lassen. So genannte Fingerpumpen erlauben durch eine sequenzielle Beaufschlagung des Fördervolumens mit verdrängenden Bauteilen die Erzeugung eines recht gleichmäßigen, linearen Förderstromes. Eine solche Anordnung ist aus DE 31 04 873 C2 bekannt, wobei auch hier der Einsatz eines Korrekturpulses vorgesehen ist.

Druckschwankungen können weiterhin durch zusätzliche technische Maßnahmen wie den Einbau von Ausgleichskolben (z. B. DD 117 108) oder eine Modifikation der Ansteuerung der verdrängenden Bauteile (z. B. DE 26 08 664 A1; GB 1 363 967) bzw. der verdrängenden Medien (z. B. Wasser in EP 0 947 207 B1) reduziert werden. Durch die Zwischenspeicherung von Teilen des Fördervolumens und dessen kontrollierter Abgabe in Phasen abfallender Drücke in der Pumpanordnung kann die Konstanz der Förderrate weiter verbessert werden. Solch eine Lösung ist u.a. für eine Kolbenpumpe aus Schrift DE 10 2004 061 813 A1 und für eine Fingerpumpe aus US 4,643,651 bekannt.

Eine Kombination des Prinzips einer Fingerpumpe und der Zwischenspeicherung von Teilmengen des zu transportierenden Mediums wird auch in den Schriften DE 39 23 457 C2 und US 5,217,355 offenbart. In beiden Schriften werden nacheinander in Flussrichtung angeordnete Bauteile verwendet, um ein zu förderndes Medium mit einer ausgangsseitig konstanten Förderrate zu transportieren. Die verwendeten Stößel übernehmen dabei zumindest eingangsseitig eine vollständige schließende Funktion. Eine Zwischenspeicherung von Teilmengen des zu fördernden Mediums wird durch die unterschiedliche Dimensionierung der einzelnen Bauteile, insbesondere derjenigen Bauteile mit Pumpfunktion erreicht. Eines dieser Bauteile mit Pumpfunktion bewirkt eine um ein ganzes Vielfaches größere Volumenänderung als das mindestens eine weitere in der Pumpanordnung vorhandene Bauteil mit Pumpfunktion. Durch eine geeignet gewählte, phasenverschobene Ansteuerung der verdrängenden Bauteile (US 5,217,355) bzw. durch die für jedes Bauteil verschiedenen Pumpzyklen (DE 39 23 457 C2) werden ausgangsseitig konstante und durch die Regulierung der Taktfrequenz der Pumpe steuerbare Förderströme erreicht. Am Eingang wird allerdings ein Stößel eingesetzt, der den Zufluss zur Pumpanordnung zeitweise unterbricht und dadurch eine stark pulsierende Förderung am Eingang der Pumpe hervorruft (siehe Phasenpläne: Fig. 4 in DE 39 23 457 C2 und Fig. 7 in US 5,217,355). In der WO 2004/076859 A2 wird eine Förderung mittels wellenförmig angesteuerter Stößel offenbart, wobei die Förderung pulsierend erfolgt.

Nachteilig an all den bisher bekannten Anordnungen zur Pulsreduktion bzw. Pulsunterdrückung sind die sehr komplizierten und teueren technischen Lösungen. Außerdem arbeiten viele bekannten Verfahren mit Ventilen, die einen Rückstrom des Mediums verhindern sollen. Solche Ventile erzeugen einen Schaltimpuls in Flussrichtung, der sich besonders in inkompressiblen Medien nachteilig fortpflanzen kann. Zudem stellen sie einen erhöhten technischen Aufwand dar. Eine pulsationsfreie Förderung wird bei allen bisher bekannten Verfahren und Vorrichtungen höchstens auf einer Seite der Pumpanordnung erreicht.

Aufgabe der Erfindung ist es, eine völlig pulsationsfreie volumetrische Förderung von Fluiden und Suspensionen mittels einer einfachen technischen Lösung zu ermöglichen.

Erfindungsgemäß wird die Aufgabe mit einem Verfahren zur pulsationsfreien volumetrischen Förderung von Fluiden und Suspensionen, mit einem aus drei Phasen bestehenden Pumpzyklus dadurch gelöst, dass während einer ersten Phase über einen Eingang eine durch die Größe einer ersten Pumpkammer definierte Volumeneinheit von außen konstant angesaugt wird, eine sich daran anschließende zweite Pumpkammer gleichen Volumens dicht geschlossen gehalten wird und mittels einer dritten Pumpkammer von der Größe des dreifachen Volumens jeder der ersten beiden Pumpkammern das gesamte Volumen in Richtung Ausgang gedrückt wird, wodurch eine vierte und fünfte Pumpkammer mit jeweils einer Volumeneinheit vollständig gefüllt werden und die dritte Volumeneinheit konstant nach außen abgegeben wird.

Im Verlauf einer zweiten Phase wird die in der ersten Phase dicht geschlossene zweite Pumpkammer vollständig geöffnet, wodurch eine Volumeneinheit durch die geöffnete erste Pumpkammer hindurch über den Eingang konstant angesaugt wird, während dessen die dritte Pumpkammer dicht geschlossen gehalten wird und die vierte Pumpkammer geschlossen wird, wodurch eine Volumeneinheit über die fünfte Pumpkammer nach außen konstant abgegeben wird.

In einer dritten Phase werden die erste und zweite Pumpkammer geschlossenen, während die dritte Pumpkammergeöffnet wird, so dass die in der ersten und zweiten Pumpkammer befindlichen Volumina sowie eine weitere Volumeneinheit über den Eingang und durch die beiden Pumpkammern hindurch von außen konstant angesaugt werden. Dadurch wird die dritte Pumpkammer mit drei Volumeneinheiten gefüllt. Gleichzeitig wird die vierte Pumpkammer dicht geschlossen gehalten. Die fünfte Pumpkammer wird während dieser Phase geschlossen, wodurch eine Volumeneinheit konstant nach außen abgegeben wird.

Die Abfolge der drei Phasen erzeugt einen konstanten Förderstrom auf der Eingangsseite der Pumpanordnung in Höhe eines definierten Volumens pro Phase. Ebenfalls steht ein konstanter Förderstrom in Höhe dieses Volumens pro Phase auf der Ausgangsseite zur Verfügung. Im Inneren der Pumpanordnung befinden sich zu jedem Zeitpunkt drei Volumina. Die dritte Pumpkammer ist so dimensioniert, dass sie die Funktion eines Zwischenspeichers erfüllt. Sie durchläuft relativ zu den anderen Pumpkammern einen Pumpzyklus, der immer dann ein Ansaugen bzw. Leerdrücken des dreifachen Volumens bewirkt, wenn sich die eingangsseitigen Pumpkammern schließen bzw. die ausgangsseitigen Pumpkammern öffnen.

Die Förderrate ist innerhalb und zwischen den jeweiligen Phasen konstant.

Eine Steuerung der Förderrate kann über die Dauer des Pumpzyklus erfolgen.

Ein besonderer Vorteil der Erfindung besteht darin, dass die Förderrichtung mit einfachen Mitteln umgekehrt werden kann. Dazu werden nur die Pumpzyklen der zweiten und vierten Pumpkammer gegeneinander ausgetauscht. Dies stimmt genau mit der zeitlichen Umkehrung aller zuvor genannten Volumenänderungen in den Kammern überein.

Bei einer besonders einfachen Ausführung wird die Ansteuerung der verdrängenden Bauteile mittels einer Nockenwelle erreicht. Eine einfache Änderung der Drehrichtung der Nockenwelle bewirkt den gegenseitigen Austausch der Pumpzyklen der zweiten und vierten Pumpkammer und führt zur Umkehr der Förderrichtung.

Erfindungsgemäß wird die Aufgabe auch mit einer Vorrichtung zur pulsationsfreien volumetrischen Förderung von Fluiden und Suspensionen mittels einer aus in Flussrichtung nacheinander angeordneten Pumpkammern bestehenden Pumpanordnung, bei der die Pumpkammern jeweils einen zeitlich gleichlangen Pumpzyklus aufweisen und bei denen die Pumpzyklen zeitlich aufeinander abgestimmt sind, dadurch gelöst, dass fünf Pumpkammern vorhanden sind und die mittlere Pumpkammer das dreifache Volumen jeder der anderen Pumpkammern aufweist. Ferner besteht der Pumpzyklus für die äußeren Pumpkammern aus drei gleichlangen Phasen und weist die Zustände der linearen Volumenzunahme über die erste Phase, der Volumenkonstanz über die zweite Phase und der linearen Volumenabnahme über die dritte Phase auf. Die mittleren drei Pumpkammern durchlaufen einen Pumpzyklus der Volumenkonstanz, der linearen Volumenzunahme und der linearen Volumenabnahme, um jeweils eine Phase versetzt und diese Pumpkammern sind im Zustand der Volumenkonstanz dicht geschlossen. Die beiden äußeren Pumpkammern durchlaufen einen identischen Pumpzyklus, weisen während der Phasen der Volumenänderung die entgegengesetzte Volumenänderung der mittleren Pumpkammer auf und sind während der Phase der Volumenkonstanz vollständig geöffnet.

Die jeweiligen Volumenänderungen je Pumpkammer werden jeweils durch die Wirkung eines verdrängenden Bauteils zwischen zwei Endzuständen, nämlich dem jeweils oberen und unteren Totpunkt des Bauteils, erzeugt.

Verdrängende Bauteile können beispielsweise Kolben, Membranen oder Stößel auf einem Schlauch sein.

Als Pumpkammer fungiert somit auch der Bereich eines rückstellfähigen Behältnisses, der sich unter einem verdrängenden Bauteil befindet. Ein solches rückstellfähiges Behältnis kann z. B. ein Schlauch sein.

Die Bewegung der verdrängenden Bauteile wird durch geeignete Ansteuerungen erzeugt und kontrolliert. Eine besonders einfache Ausführung ist die Verwendung einer Nockenwelle, ferner sind piezoelektrische Aktoren oder Stellmotoren möglich. Die Ansteuerung kann dabei durch formschlüssig mechanische, magnetische, pneumatische oder hydraulische Einwirkungen realisiert sein.

Die Erfindung soll nachstehend anhand von Ausführungsbeispielen näher erläutert werden. Die dazugehörigen Zeichnungen zeigen:

Fig. 1: den Phasenplan des Verfahrens;

Fig. 2: eine schematische Darstellung einer erfindungsgemäßen Pump□anordnung;

Fig. 3: eine schematische Darstellung einer weiteren erfindungsgemäßen Pumpanordnung;

Fig. 4: eine schematische Darstellung einer weiteren erfindungsgemäßen Pumpanordnung;

Fig. 5: eine schematische Darstellung einer erfindungsgemäßen Schlauch□pumpe.

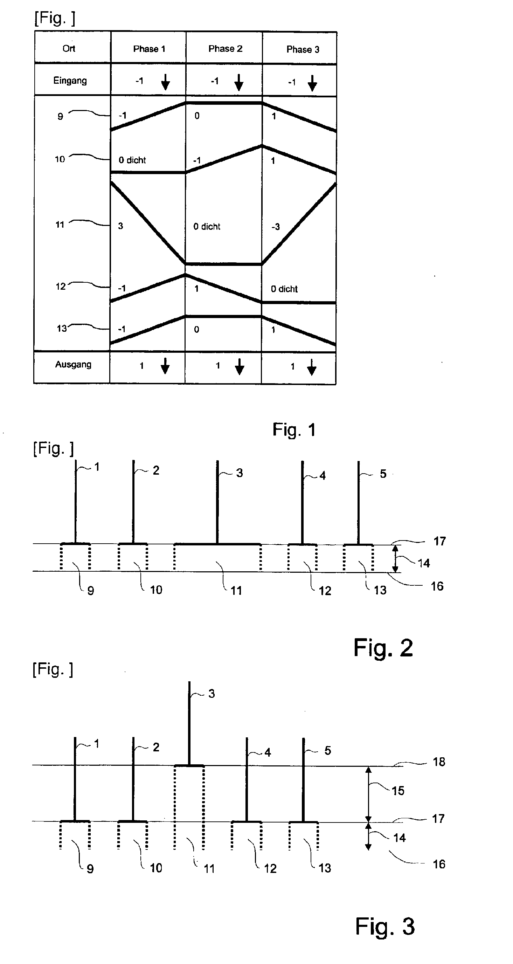

Die durch eine Ansteuerung 8 hervorgerufene Bewegung der verdrängenden Bauteile 1 bis 5 führen in den Pumpkammern 9 bis 13 zu den drei Zuständen der linearen Volumenzunahme, der Volumenkonstanz und der linearen Volumenabnahme (Fig. 1). Die beiden Zustände der Volumenkonstanz treten entweder bei geschlossener Pumpkammer (unten liegende Linien in Fig. 1) oder bei vollständig geöffneter Pumpkammer (oben liegenden Linien in Fig. 1) auf.

Erfindungsgemäß stehen die in den einzelnen Pumpkammern angesaugten bzw. abgedrückten Volumina in einem definierten zahlenmäßigen Verhältnis zueinander, wie in Fig. 1 als bewegte relative Volumina angegeben. In der ersten, zweiten, vierten und fünften Pumpkammer wird je Phase jeweils ein Volumen angesaugt bzw. abgedrückt. In der dritten Pumpkammer werden in Phasen mit Volumenänderungen drei Volumen bewegt.

In den einzelnen Pumpkammern bewirken Saughübe (negative Vorzeichen in Fig. 1) eine Förderung des Mediums aus Richtung des Eingangs der Pumpanordnung in eine Pumpkammer hinein, während Druckhübe (positive Vorzeichen in Fig. 1) das Medium aus einer Pumpkammer hinaus in Richtung des Ausgangs der Pumpanordnung fördern.

Über die erste Phase des Verfahrens öffnet sich die erste Pumpkammer 9 gleichmäßig und saugt am Eingang der Pumpanordnung ein Volumen von außen an. Da die zweite Pumpkammer 10 über die ganze erste Phase dicht geschlossen und völlig entleert ist, kann von dort nicht angesaugt werden. Am Ende der ersten Phase wurde eine Volumeneinheit in die Pumpanordnung eingesaugt, die sich nunmehr in der ersten Pumpkammer 9 befindet.

Über die zweite Phase bleibt die erste Pumpkammer 9 vollständig geöffnet. Das bedeutet, dass sich das Volumen der ersten Pumpkammer 9 über diese Phase nicht ändert. Allerdings kann Medium durch die erste Pumpkammer 9 hindurch strömen. Dies geschieht, da sich die zweite Pumpkammer 10 über die zweite Phase gleichmäßig öffnet, wodurch eine Volumeneinheit durch die erste, geöffnete Pumpkammer 9 hindurch von außen angesaugt wird. Die dritte Pumpkammer 11 ist derweil dicht geschlossen und sperrt die Pumpanordnung in Förderrichtung ab. Am Ende der zweiten Phase wurden in die Pumpanordnung kontinuierlich bereits zwei Volumeneinheiten angesaugt, wobei die erste und die zweite Pumpkammer 9, 10 mit jeweils einer Volumeneinheit gefüllt sind.

Über die dritte Phase öffnet sich nun die dritte Pumpkammer 11. Diese saugt ein Volumen an, das dreimal so groß ist wie die jeweiligen Volumen der ersten und zweiten Pumpkammer 9, 10. Zwei der drei Volumen stammen aus den sich über die dritte Phase schließenden ersten und zweiten, mit jeweils einem Volumen gefüllten, Pumpkammern 9, 10. Das dritte Volumen wird von außen durch die erste und zweite Pumpkammer 9, 10 hindurch angesaugt. Über die dritte Phase ist die vierte Pumpkammer 12 dicht geschlossen und erlaubt daher nur ein eingangsseitiges Ansaugen. Zum Ende der dritten Phase, d. h. über einen kompletten Pumpzyklus, wurden kontinuierlich drei Volumeneinheiten eingangsseitig in die Pumpanordnung angesaugt. Dabei sind nun die erste und zweite Pumpkammer 9, 10 leer, während die dritte Pumpkammer 11 mit drei Volumeneinheiten gefüllt ist.

Ausgangsseitig wird über die erste Phase eines Pumpzyklus eine Volumeneinheit kontinuierlich nach außen abgeben. Dies wird dadurch bewirkt, dass sich die bereits gefüllte dritte Pumpkammer 11 schließt und dabei die in ihr enthaltenen drei Volumina in Richtung der vierten und fünften Pumpkammer 12, 13 gedrückt werden. Ein Rückfluss in Richtung des Eingangs ist nicht möglich, da die zweite Pumpkammer 10 über die erste Phase dicht geschlossen ist. Zwei der von der dritten Pumpkammer 11 abgedrückten drei Volumina werden von den sich gleichzeitig öffnenden vierten und fünften Pumpkammern 12, 13 aufgenommen. Das dritte Volumen wird durch die vierte und fünfte Pumpkammer 12, 13hindurch kontinuierlich am Ausgang abgegeben. Zum Ende der ersten Phase ist die dritte Pumpkammer 11 völlig entleert, während die vierte und fünfte Pumpkammer 12, 13 mit jeweils einem Volumen gefüllt sind.

Über die zweite Phase bleibt die entleerte dritte Pumpkammer 11 dicht geschlossen. Die sich in dieser Phase schließende vierte Pumpkammer 12 kann das in ihr beinhaltete eine Volumen daher nur in Richtung der fünften Pumpkammer 13 drücken. Die fünfte Pumpkammer 13 ist über die Phase gefüllt und vollständig geöffnet. Das aus der vierten Pumpkammer 12 abgedrückte eine Volumen gelangt durch die fünfte Pumpkammer 13 hindurch kontinuierlich nach außen. Am Ende der zweiten Phase eines Pumpzyklus wurden somit bereits zwei Volumeneinheiten am Ausgang der Pumpanordnung abgegeben. Dabei ist die vierte Pumpkammer 12 entleert während die fünfte Pumpkammer 13 geöffnet und mit einem Volumen gefüllt ist.

Über die dritte Phase bleibt nun die vierte Pumpkammer 12 dicht geschlossen. Dadurch kann das durch die sich schließende fünfte Pumpkammer 13 abgedrückte eine Volumen nur nach außen hin abgegeben werden. Zum Ende der dritten Phase ist die fünfte Pumpkammer 13 entleert.

Am Ende der dritten Phase ist ein Pumpzyklus abgeschlossen, so dass sich alle Pumpkammern (9 bis 13) der Pumpanordnung in einem Zustand befinden, der den unmittelbaren Wiedereintritt in die erste Phase eines neuen Pumpzyklus erlaubt. Wie soeben beschrieben wird somit über einen Pumpzyklus hindurch ständig ein definiertes Volumen eingangsseitig kontinuierlich angesaugt und gleichzeitig ein gleichgroßes Volumen kontinuierlich ausgangsseitig abgegeben.

Die Wirkung der zweiten und vierten Pumpkammer 10, 12 beruht auf der Sperrung einer Richtung durch dichtes Schließen immer dann, wenn die dritte Pumpkammer 11 ihre drei Volumina abdrückt bzw. ansaugt. Dadurch bestimmen die zweite und vierte Pumpkammer 10, 12 die Förderrichtung in der ersten und dritten Phase (Fig. 1). Über die zweite Phase bewirken allein die Pumpzyklen der zweiten und vierten Pumpkammer 10, 12 das eingangsseitige Ansaugen und das ausgangsseitige Abdrücken eines Volumens. Alle anderen Pumpkammern 9, 11,13 sind über die zweite Phase im Zustand der Volumenkonstanz. Die Richtung der Förderung wird daher in allen Phasen ausschließlich durch die Wirkung der Pumpzyklen der zweiten und vierten Pumpkammer 10, 12 bestimmt. Ein gegenseitiger Austausch der Pumpzyklen der zweiten und vierten Pumpkammer 10, 12 führt daher zu einer Umkehr der Förderrichtung.

In einer ersten vorteilhaften Ausgestaltung gemäß Fig. 2 weist das dritte verdrängende Bauteil 3 die dreifache verdrängende Oberfläche wie jedes der anderen verdrängenden Bauteile auf. Alle verdrängenden Bauteile durchlaufen in Phasen mit einer Volumenänderung die gleiche einfache Hublänge 14 zwischen ihrem oberen Totpunkt 17 und ihrem unteren Totpunkt 16.

In einer zweiten vorteilhaften Ausführung gemäß Fig. 3 weisen alle verdrängenden Bauteile 1 bis 5 die gleiche verdrängende Oberfläche auf, allerdings durchläuft das dritte verdrängende Bauteil 3 in Phasen mit Volumenänderung eine im Verhältnis zu den anderen verdrängenden Bauteilen 1, 2, 4 und 5 dreifache Hublänge 15.

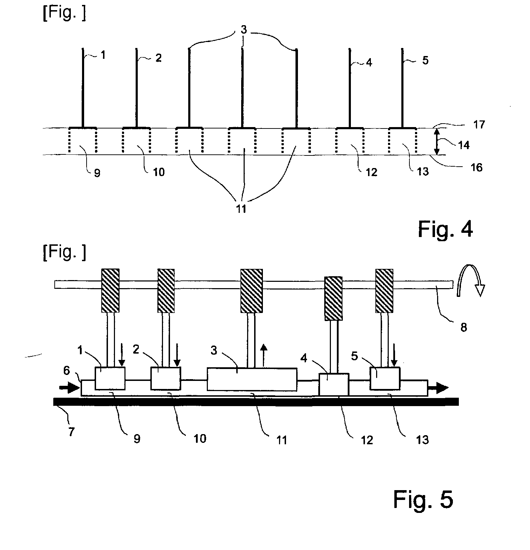

In einer dritten vorteilhaften Ausgestaltung gemäß Fig. 4 wird das dritte verdrängende Bauteil 3 durch drei identische verdrängende Bauteile ersetzt, deren verdrängende Oberfläche sowie die in Phasen der Volumenänderung zu durchlaufende Hublänge 15 identisch zur verdrängenden Oberfläche sowie in Phasen der Volumenänderung zu durchlaufenden Hublänge 14 der anderen verdrängenden Bauteilen 1, 2, 4, 5 in der ersten, zweiten, vierten und fünften Pumpkammer 9, 10, 12, 13 sind. Wird das dritte verdrängende Bauteil 3 durch drei identische verdrängende Bauteile ersetzt, müssen sich diese dann identisch und im Pumpzyklus desjenigen verdrängenden Bauteils 3 bewegen, welches sie ersetzen. Dadurch können identische Pumpkammern zur Durchführung des Verfahrens eingesetzt werden.

In einen Ausführungsbeispiel gemäß Fig. 5 sind die verdrängenden Bauteile 1 bis 5 als fünf Stößel ausgebildet, die über eine, durch eine Nockenwelle realisierte, Ansteuerung 8 einen auf einer Druckplatte 7 befindlichen Schlauch 6 beaufschlagen. Diejenigen Bereiche innerhalb des Schlauches 6, die sich unter den Stößeln 1 bis 5 befinden, fungieren dabei als Pumpkammern 9 bis 13. Eine solche Ausgestaltung hat den Vorteil, dass das geförderte Medium nicht mit Teilen des Pumpmechanismus in Kontakt kommt und eine sterile Förderung gewährleistet wird. Zudem entfällt die Reinigung des Pumpmechanismus nach Benutzung oder bei einem Wechsel des zu fördernden Mediums. Ein Austausch des Schlauches 6 ist einfach zu realisieren und reicht vollkommen aus.

In weiteren Ausgestaltungen kann jede der Pumpkammern 9 bis 13 durch kleinere Pumpkammern ersetzt werden, die hinsichtlich ihrer Volumina, Form und Anzahl beliebig ausgestaltet werden können, solange sie einen identischen Pumpzyklus wie diejenige Pumpkammer 9 bis 13, die durch die kleineren Pumpkammern ersetzt wurde, durchlaufen. Zudem muss die Summe der Volumina der kleineren Pumpkammern mit dem Volumen derjenigen Pumpkammer 9 bis 13 übereinstimmen, die durch die kleineren Pumpkammern ersetzt wurde.

In einer weiteren Ausführung können sowohl die erste als auch die fünfte Pumpkammer 9, 13frei gestaltet werden und jeweils ein Volumen aufweisen, das größer ist als die jeweiligen Volumina der zweiten und vierten Pumpkammer 10, 12. Dabei ist dann zu beachten, dass je Phase nur genau das Volumen in der ersten und fünften Pumpkammer 9, 13angesaugt bzw. abgedrückt wird, das der jeweiligen Größe der zweiten und vierten Pumpkammer 10, 12entspricht.

Die Forderung nach dem Einsatz von Pumpen, die weder am Ein- noch am Ausgang pulsierende Förderströme aufweisen, stellt sich z. B. beim Betrieb volumenstarrer Systeme, bei denen sich auch eingangseitige Pulse dem gesamten Kreislauf mitteilen können. Weiterhin sind vollkommen pulsationsfreie Pumpen bei solchen Anwendungen höchst sinnvoll einzusetzen, bei denen sowohl vor als auch nach der Pumpe Pulse vermieden werden sollen um unangenehme oder pathologische Wirkungen (z. B. bei intravenösen Infusionen, Dialyse), messtechnische Probleme (z. B. bei der Überwachung des Volumenstromes) oder Veränderungen des zu transportierenden Mediums (z.B. Entmischung von Fluiden) zu verhindern. Das erfindungsgemäße Verfahren und die Vorrichtung sind sowohl für große Laboranwendungen wie der Umwälzung von Zell-Suspensionen in geschlossenen Fermentern als auch für die pulsationsfreie und konstante Förderung sehr kleiner Mengen geeignet, wie sie z. B. in gentechnischen Verfahren erforderlich sind. Weiterhin ist es für viele Anwendungen äußerst vorteilhaft, dass die Förderrichtung der Pumpanordnung nach dem erfindungsgemäßen Verfahren einfach umgekehrt werden kann.

### Bezugszeichenliste

- 1.: erstes verdrängendes Bauteil
- 2.: zweites verdrängendes Bauteil
- 3.: drittes verdrängendes Bauteil
- 4.: viertes verdrängendes Bauteil
- 5.: fünftes verdrängendes Bauteil
- 6.: Schlauch
- 7.: Druckplatte
- 8.: Ansteuerung der verdrängenden Bauteile
- 9.: erste Pumpkammer
- 10.: zweite Pumpkammer
- 11.: dritte Pumpkammer
- 12.: vierte Pumpkammer
- 13.: fünfte Pumpkammer
- 14.: einfache Hublänge
- 15.: dreifache Hublänge
- 16.: unterer Totpunkt aller verdrängenden Bauteile
- 17.: oberer Totpunkt der verdrängenden Bauteile 1, 2, 4, 5
- 18.: oberer Totpunkt des verdrängenden Bauteils 3

## Patentansprüche

1. Verfahren zur pulsationsfreien volumetrischen Förderung von Fluiden und Suspensionen, mit einem Pumpzyklus der aus drei Phasen besteht, wobei
während einer ersten Phase wird über einen Eingang eine durch die Größe einer ersten Pumpkammer (9) definierte Volumeneinheit von außen konstant ansaugt, eine sich daran anschließende zweite Pumpkammer (10) gleichen Volumens wird dicht geschlossen gehalten und mittels einer dritten Pumpkammer (11) von der Größe des dreifachen Volumens wird das gesamte Volumen in Richtung Ausgang gedrückt, wodurch eine vierte (12) und fünfte (13) Pumpkammer mit jeweils einer Volumeneinheit gefüllt werden und die dritte Volumeneinheit konstant nach außen abgegeben wird,
während einer zweiten Phase wird die in der ersten Phase dicht geschlossene zweite Pumpkammer (10) vollständig geöffnet, wodurch eine Volumeneinheit durch die geöffnete erste Pumpkammer (9) hindurch über den Eingang konstant angesaugt wird, während dessen wird die dritte Pumpkammer (11) dicht geschlossen gehalten und die vierte Pumpkammer (12) wird geschlossen, wodurch eine Volumeneinheit über die fünfte Pumpkammer (13) nach außen konstant abgegeben wird,
während einer dritten Phase werden die erste (9) und zweite (10) Pumpkammer geschlossenen, während dessen die dritte Pumpkammer (11) geöffnet wird, so dass die Volumen der ersten (9) und zweiten (10) Pumpkammer sowie eine weitere Volumeneinheit über den Eingang und die beiden Pumpkammern (9), (10) hindurch von außen konstant angesaugt werden und dadurch die dritte Pumpkammer (11) mit drei Volumeneinheiten gefüllt ist, gleichzeitig wird die vierte Pumpkammer (12) dicht geschlossen gehalten und die fünfte Pumpkammer (13) wird geschlossen, wodurch eine Volumeneinheit nach außen konstant abgegeben wird.

2. Verfahren nach Anspruch 1, **dadurch gekennzeichnet, dass** alle drei Phasen gleichlang gestaltet werden.

3. Verfahren nach Anspruch 1 oder 2, **dadurch gekennzeichnet, dass** alle Volumenänderungen zeitlich linear erfolgen.

4. Verfahren nach einem der vorhergehenden Ansprüche, **dadurch gekennzeichnet dass** durch einen gegenseitigen Austausch der Arbeitsweisen der zweiten (10) und vierten (12) Pumpkammer in jeder Phase eine Umkehr der Flussrichtung erreicht wird.

5. Vorrichtung zur pulsationsfreien volumetrischen Förderung von Fluiden und Suspensionen mittels einer Pumpanordnung bestehend aus in Flussrichting nacheinander angeordneten und miteinander verbundenen Pumpkammern die jeweils einen zeitlich gleichlangen Pumpzyklus aufweisen und bei denen die Pumpzyklen zeitlich aufeinander abgestimmt sind, **dadurch gekennzeichnet, dass** fünf Pumpkammern (9 bis 13) vorhanden sind,
die mittlere Pumpkammer (11) das dreifache Volumen jeder der anderen Pumpkammern (9,10, 12, 13) aufweist,
der Pumpzyklus jeweils aus drei gleichlangen Phasen besteht, wobei über eine Phase der Zustand der linearen Volumenzunahme, über eine weitere Phase der Zustand der Volumenkonstanz und über eine noch weitere Phase der Zustand der linearen Volumenabnahme einnehmbar ist,
die mittleren drei Pumpkammern (10 bis 12) jeweils nacheinander jedoch um eine Phase versetzt, die Phasen der Volumenkonstanz, der linearen Volumenzunahme und der linearen Volumenabgabe aufweisen und die Pumpkammern (10 bis 12) im Zustand der Volumenkonstanz dicht geschlossen sind,
die beiden äußeren Pumpkammern (9, 13) einen identischen Pumpzyklus mit den Phasen lineare Volumenzunahme, der Volumenkonstanz und der linearen Volumenabgabe aufweisen,
die beiden äußeren Pumpkammern (9, 13) während der Phasen der Volumenänderung die entgegengesetzte Volumenänderung der mittleren Pumpkammer (10) aufweisen und während der Phase der Volumenkonstanz vollständig geöffnet sind.

6. Vorrichtung nach Anspruch 5, **dadurch gekennzeichnet, dass** ein verdrängendes Bauteil (3) in der mittleren Pumpkammer (11) die gleiche Oberfläche wie die verdrängenden Bauteile (1, 2, 4, 5) in den anderen Pumpkammer (9, 10, 12, 13) aufweist, aber während jeder Phase mit Volumenänderung eine Hublänge (15) durchläuft, die dreimal so groß ist wie die Hublänge (14) jedes der andern verdrängenden Bauteile (1, 2, 4, 5).

7. Vorrichtung nach Anspruch 5, **dadurch gekennzeichnet, dass** die dritte Pumpkammer (11) durch drei identische Pumpkammern mit einfachem Volumen ersetzt ist, die gleichzeitig einen identischen Pumpzyklus durchlaufen.

8. Vorrichtung nach Anspruch 5, **dadurch gekennzeichnet, dass** jede einzelne Pumpkammer (9 bis 13) aus einer beliebigen Anzahl von kleineren Pumpkammern mit gleichen oder unterschiedlichen Volumina zusammengesetzt ist, wobei die jeweils zu einer einzelnen Pumpkammer zusammengehörigen kleineren Pumpkammern alle gleichzeitig den identischen Pumpzyklus durchlaufen und die Summe der Volumina der kleineren Pumpkammern dem Volumen derjenigen Pumpkammer entspricht, die durch die kleineren Pumpkammern ersetzt sind.

9. Vorrichtung nach Anspruch 5, **dadurch gekennzeichnet, dass** ein rückstellfähiges Behältnis durch verdrängende Bauteile (1 bis 5) beaufschlagt wird und dadurch unter jedem verdrängenden Bauteil (1 bis 5) ein als Pumpkammer (9 bis 13) wirkender Bereich vorhanden ist.

10. Vorrichtung nach Anspruch 9, **dadurch gekennzeichnet, dass** das rückstellfähige Behältnis ein Schlauch (6) ist.

11. Vorrichtung nach Anspruch 9 oder 10, **dadurch gekennzeichnet, dass** das verdrängende Bauteil ein Stößel ist.

12. Vorrichtung nach Anspruch 11, **dadurch gekennzeichnet, dass** der mittlere Stößel dreimal soviel Volumen verdrängt, wie jeder der anderen Stößel allein.

13. Vorrichtung nach Anspruch 11, **dadurch gekennzeichnet, dass** der mittlere Stößel durch drei identische Stößel ersetzt ist, die gleichzeitig einen identischen Pumpzyklus realisieren.

14. Vorrichtung nach Anspruch 11, **dadurch gekennzeichnet, dass** jeder einzelne Stößel durch eine beliebige Anzahl von kleineren Stößeln ersetzt ist, wobei die jeweils zu einem einzelnen Stößel zusammengehörigen kleineren Stößel alle gleichzeitig den identischen Pumpzyklus durchlaufen und die Summe der durch die kleineren Stößel verdrängten Volumina dem jeweils mittels dem ersetzten Stößel verdrängten Volumen entspricht.

15. Vorrichtung nach Anspruch 5, **dadurch gekennzeichnet, dass** die erste und / oder die fünfte Pumpkammer (9, 13) jeweils ein größeres Volumen als die zweite und vierte Pumpkammer (10, 12) aufweisen, wobei der Hubraum der ersten und / oder fünften Pumpkammer (9, 13) dem jeweiligen Volumen der zweiten und vierten Pumpkammer (10, 12)entspricht.

## Claims

1. A method for the pulsation-free volumetric delivery of fluids and suspensions, with a pump cycle consisting of three phases, wherein
during a first phase, a unit volume defined by the size of a first pump chamber (9) is continuously taken in from outside via an inlet, an attached second pump chamber (10) of the same volume is kept tightly closed and, by way of a third pump chamber (11) of the size of three times the volume, the whole volume is pressed towards an outlet, whereby a fourth (12) and a fifth (13) pump chamber are each filled with one unit volume and the third unit volume is continuously put out,
during a second phase, the second pump chamber (10) which is tightly closed in the first phase is opened completely, whereby a unit volume is continuously taken in through the open first pump chamber (9) via the inlet, while the third pump chamber (11) is kept tightly closed and the fourth pump chamber (12) is closed, whereby a unit volume is continuously put out via the fifth pump chamber (13),
during a third phase, the first (9) and second (10) pump chamber are closed, while the third pump chamber (11) is opened so that the volumes of the first (9) and second (10) pump chamber as well as another unit volume are continuously taken in from outside via the inlet and through the two pump chambers (9), (10) and that the third pump chamber (11) is thereby filled with three unit volumes, simultaneously the fourth pump chamber (12) is kept tightly closed and the fifth pump chamber (13) is closed, whereby a unit volume is continuously put out.

2. A method according to claim 1, **characterized in that** all three phases have the same length.

3. A method according to claim 1 or 2, **characterized in that** all volume changes are linear in time.

4. A method according to any of the preceding claims, **characterized in that** by a mutual exchange of the functioning of the second (10) and fourth (12) pump chamber a reversal of the flow direction is achieved in every phase.

5. An apparatus for the pulsation-free volumetric delivery of fluids and suspensions by way of a pump configuration consisting of pump chambers consecutively arranged in the flow direction and connected to each other, which each have a pump cycle of the same temporal length and wherein the pump cycles are synchronized in time, **characterized in that**
there are five pump chambers (9 to 13),
the central pump chamber (11) has three times the volume of each of the other pump chambers (9, 10, 12, 13),
the pump cycle consists of three phases each of the same length, wherein by one phase the state of linear volume increase, by another phase the state of volume constancy and by yet another phase the state of linear volume decrease can be assumed,
the three central pump chambers (10 to 12), each consecutively but shifted by one phase, have the phases of volume constancy, of linear volume increase and of linear volume decrease and the pump chambers (10 to 12) are tightly closed in the state of volume constancy,
the two outer pump chambers (9, 13) have an identical pump cycle with the phases of linear volume increase, of volume constancy and of linear volume decrease,
the two outer pump chambers (9, 13) show the opposite volume change of the central pump chamber (10) during the phases of volume change and are completely open during the phase of volume constancy.

6. An apparatus according to claim 5, **characterized in that** a displacing component (3) in the central pump chamber (11) has the same surface as the displacing components (1, 2, 4, 5) in the other pump chambers (9, 10, 12, 13), but passes a stroke length (15), which is three times as long as the stroke length (14) of each of the other displacing components (1, 2, 4, 5), during each phase, in which a volume change occurs.

7. An apparatus according to claim 5, **characterized in that** the third pump chamber (11) is replaced by three identical pump chambers of simple volume, which simultaneously undergo an identical pump cycle.

8. An apparatus according to claim 5, **characterized in that** every single pump chamber (9 to 13) is composed of an unlimited number of smaller pump chambers of the same or different volume, wherein the smaller pump chambers belonging to a single pump chamber all simultaneously undergo the identical pump cycle and the sum of the volumes of the smaller pump chambers corresponds to the volume of the pump chamber, which is replaced by the smaller pump chambers.

9. An apparatus according to claim 5, **characterized in that** the displacing components (1 to 5) act on an elastic container, whereby a section of the elastic container underneath each displacing component (1 to 5) functions as pump chamber (9 to 13).

10. An apparatus according to claim 9, **characterized in that** the elastic container is a tube (6).

11. An apparatus according to claim 9 or 10, **characterized in that** the displacing component is a plunger.

12. An apparatus according to claim 11, **characterized in that** the central plunger displaces three times as much volume as each of the other plungers alone.

13. An apparatus according to claim 11, **characterized in that** the central plunger is replaced by three identical plungers, which simultaneously realize an identical pump cycle.

14. An apparatus according to claim 11, **characterized in that** every single plunger is replaced by an unlimited number of smaller plungers, wherein the smaller plungers belonging to a single plunger all simultaneously undergo the identical pump cycle and the sum of the volumes displaced by the smaller plungers corresponds to the volume displaced by the replaced plunger.

15. An apparatus according to claim 5, **characterized in that** the first and / or the fifth pump chamber (9, 13) each have a larger volume than the second and the fourth pump chamber (10, 12), wherein the swept volume of the first and / or fifth pump chamber (9, 13) corresponds to the respective volume of the second and fourth pump chamber (10, 12).

## Revendications

1. Procédé pour le transport volumétrique sans pulsation de fluides et suspensions, avec un cycle de pompage composé de trois phases, dans lequel pendant une première phase, une unité de volume définie par la taille d'une première chambre de pompage (9) est constamment aspirée de l'extérieur à travers une entrée, une deuxième chambre de pompage (10) de même volume, qui s'y raccorde, est tenue bien fermée et, par une troisième chambre de pompage (11) de la taille du triple volume, tout le volume est poussé vers une sortie, de sorte qu'une quatrième (12) et cinquième (13) chambre de pompage sont chacune remplies d'une unité de volume et la troisième unité de volume est constamment évacuée à l'extérieur,
pendant une deuxième phase, la deuxième chambre de pompage (10), qui est bien fermée pendant la première phase, est ouverte complètement, de sorte qu'une unité de volume est constamment aspirée par la première chambre de pompage (9) ouverte à travers l'entrée, pendant que la troisième chambre de pompage (11) est tenue bien fermée et la quatrième chambre de pompage (12) est fermée, de sorte qu'une unité de volume est constamment évacuée à l'extérieur à travers la cinquième chambre de pompage (13),
pendant une troisième phase, la première (9) et deuxième (10) chambre de pompage sont fermées, pendant que la troisième chambre de pompage (11) est ouverte, de sorte que les volumes de la première (9) et deuxième (10) chambre de pompage ainsi qu'une autre unité de volume sont constamment aspirés de l'extérieur à travers l'entrée et les deux chambres de pompage (9), (10) et par là, la troisième chambre de pompage (11) est remplie de trois unités de volume, simultanément la quatrième chambre de pompage (12) est tenue bien fermée et la cinquième chambre de pompage (13) est fermée, de sorte qu'une unité de volume est constamment aspirée à l'extérieur.

2. Procédé selon la revendication 1, **caractérisé en ce que** les trois phases ont la même longueur.

3. Procédé selon la revendication 1 ou 2, **caractérisé en ce que** tous les changements volumétriques sont linéaires dans le temps.

4. Procédé selon une des revendications précédentes, **caractérisé en ce que** par un échange mutuel des fonctionnements de la deuxième (10) et quatrième (12) chambre de pompage une inversion du sens de flux est obtenue dans chaque phase.

5. Dispositif pour le transport volumétrique sans pulsation de fluides et suspensions par une configuration de pompage composée de chambres de pompage liées et disposées l'une après l'autre en sens de flux, chacune ayant un cycle de pompage de même longueur temporelle et les cycles de pompage étant synchronisés dans le temps, **caractérisé en ce que**
il y a cinq chambres de pompage (9 à 13),
la chambre de pompage centrale (11) a le triple volume de chacune des autres chambres de pompage (9, 10, 12, 13),
chaque cycle de pompage est composé de trois phases de même longueur, dans lequel par une phase l'état d'augmentation volumétrique linéaire, par une autre phase l'état de constance volumétrique et par encore une autre phase l'état de réduction volumétrique linéaire peut être adopté,
les trois chambres de pompage centrales (10 à 12), chacune successivement mais décalée d'une phase, ont les phases de constance volumétrique, d'augmentation volumétrique linéaire et de réduction volumétrique linéaire, et les chambres de pompage (10 à 12) sont bien fermées dans l'état de constance volumétrique,
les deux chambres de pompage extérieures (9, 13) ont un cycle de pompage identique avec les phases d'augmentation volumétrique linéaire, de constance volumétrique et de réduction volumétrique linéaire, les deux chambres de pompage extérieures (9, 13) ont le changement volumétrique opposé de la chambre de pompage centrale (10) pendant les phases de changement volumétrique et sont complètement ouvertes pendant la phase de constance volumétrique.

6. Dispositif selon la revendication 5, **caractérisé en ce qu'**un composant déplaçant (3) dans la chambre de pompage centrale (11) a le même surface que les composants déplaçants (1, 2, 4, 5) dans les autres chambres de pompage (9, 10, 12, 13), mais passe une longueur de course (15) trois fois plus longue que la longueur de course (14) de chacun des autres composants déplaçants (1, 2, 4, 5), pendant chaque phase d'un changement volumétrique.

7. Dispositif selon la revendication 5, **caractérisé en ce que** la troisième chambre de pompage (11) est remplacée par trois chambres de pompage identiques de volume simple, qui simultanément accomplissent un cycle de pompage identique.

8. Dispositif selon la revendication 5, **caractérisé en ce que** chaque chambre de pompage (9 à 13) est composée d'un nombre illimité de plus petites chambres de pompage de même ou différent volume, de sorte que les plus petites chambres de pompage, qui appartiennent à une chambre de pompage particulière, toutes simultanément accomplissent le cycle de pompage identique et la somme des volumes des plus petites chambres de pompage correspond au volume de la chambre de pompage qui est remplacée par les plus petites chambres de pompage.

9. Dispositif selon la revendication 5, **caractérisé en ce que** des composants déplaçants (1 à 5) agissent sur un récipient élastique, de sorte qu'une section du récipient élastique sous chaque composant déplaçant (1 à 5) fonctionne comme chambre de pompage (9 à 13)

10. Dispositif selon la revendication 9, **caractérisé en ce que** le récipient élastique est un tuyau (6).

11. Dispositif selon la revendication 9 ou 10, **caractérisé en ce que** le composant déplaçant est un poussoir.

12. Dispositif selon la revendication 11, **caractérisé en ce que** le poussoir central déplace trois fois plus de volume que chacun des autres poussoirs seuls.

13. Dispositif selon la revendication 11, **caractérisé en ce que** le poussoir central est remplacé par trois poussoirs identiques, qui simultanément réalisent un cycle de pompage identique.

14. Dispositif selon la revendication 11, **caractérisé en ce que** chaque poussoir est remplacé par un nombre illimité de plus petits poussoirs, dans lequel les plus petits poussoirs, qui appartiennent à un poussoir particulier, tous simultanément accomplissent le cycle de pompage identique et la somme des volumes déplacés par les plus petits poussoirs correspond au volume déplacé par le poussoir remplacé.

15. Dispositif selon la revendication 5, **caractérisé en ce que** la première et / ou la cinquième chambre de pompage (9, 13) chacune ont un plus grand volume que la deuxième et quatrième chambre de pompage (10, 12), dans lequel la cylindrée de la première et / ou cinquième chambre de pompage (9, 13) correspond au volume respectif de la deuxième et quatrième chambre de pompage (10, 12).
